# EUROPÄISCHE PATENTSCHRIFT

(11) **EP 0 716 599 B1**
(45) Veröffentlichungstag und Bekanntmachung des Hinweises auf die Patenterteilung: **01.04.1998**
(21) Anmeldenummer: 93923559.4
(22) Anmeldetag: 27.10.1993
(51) Int. Cl.: A61K 9/70

(54) **TRANSDERMALES THERAPEUTISCHES SYSTEM ZUR ABGABE VON 17-$g(b)-ESTRADIOL UND VERFAHREN ZU SEINER HERSTELLUNG**
TRANSDERMAL THERAPEUTIC SYSTEM FOR THE RELEASE OF 17-$g(b)-ESTRADIOL AND PROCESS FOR ITS PRODUCTION
SYSTEME THERAPEUTIQUE TRANSDERMIQUE POUR L'APPLICATION DE 17-$g(b)- STRADIOL ET SON PROCEDE DE FABRICATION

(30) Priorität: 06.11.1992 DE 4237453
(43) Veröffentlichungstag der Anmeldung: 19.06.1996
(73) Patentinhaber: LTS LOHMANN Therapie-Systeme GmbH, 56567 Neuwied (DE)
(72) Erfinder: HORSTMANN, Michael, D-56564 Neuwied (DE); HAHN, Sylvia, D-56170 Bendorf (DE); MECONI, Reinhold, D-56567 Neuwied (DE); KLEIN, Robert-Peter, D-56567 Neuwied (DE)
(74) Vertreter: Flaccus, Rolf-Dieter, Dr.
(86) Internationale Anmeldenummer: EP9302971
(87) Internationale Veröffentlichungsnummer: WO9410984

(56) Entgegenhaltungen:
- EP-A- 0 379 045
- EP-A- 0 413 034
- EP-A- 0 421 454
- EP-A- 0 430 491
- EP-A- 0 439 180
- EP-A- 0 483 370
- WO-A-90/06736
- WO-A-90/10425
- WO-A-91/05529
- FR-A- 2 527 450
- FR-A- 2 612 785

## Beschreibung

Die Erfindung betrifft ein transdermales therapeutisches System zur Abgabe von 17-β-Estradiol und gegebenenfalls weiterer Wirkstoffe über die Haut an den menschlichen Körper.

Transdermale therapeutische Systeme (TTS) sind in der arzneilichen Therapie einer Reihe von Erkrankungen bereits im Markt eingeführt.
Auch TTS mit dem Wirkstoff 17-β-Estradiol sind inzwischen als Therapeutikum für Wechseljahrsbeschwerden, neuerdings auch gegen Osteoporose in Handel und in der Therapie erfolgreich. Die in EP 0 421 454 beschriebenen Systeme enthalten Estradiol in einem wasserquellbaren Acrylatpolymer unter Zusatz von "Kristallisationshemmern" und klebrigmachenden Harzen. Die Beeinflussung der Löslichkeit von Estradiol durch unterschiedliche Wasserdampfspannung wurde jedoch nicht erkannt und demzufolge findet sich auch nicht die Lehre für die korrekte Einstellung der Konzentration von Estradiol.

Ein Nachteil, der dem Stand der Technik entsprechenden Systeme ist die Notwendigkeit, sogenannte "Enhancer" verwenden zu müssen. Es handelt sich hier um in der Regel flüssige Zusatzstoffe, die die Resorptionseigenschaften der menschlichen Haut verbessern und damit auch die Aufnahme des Wirkstoffs Estradiol aus einer genügend kleinen TTS-Fläche heraus ermöglichen. Speziell die leicht flüchtigen Enhancer wie das im Falle von Estradiol regelmäßig verwendete Ethanol birgt Probleme der allzu starken Erweichung der Klebschichten von TTS und macht vor allem weitere, raumfordernde Kompartimente im System notwendig, die das TTS unakzeptabel großflächig bzw. dick werden lassen.

Unter Zusatz bestimmter weniger flüchtiger, meist aber auch weniger aktiver Enhancer ist zwar die Herstellung von Matrixsystemen möglich, die den Wirkstoff und die resorptionsverstärkende Komponente in einer oder wenigen monolithischen Schichten enthalten.
US 4,863,738 stellt so ein Bespiel von vielen dar, in denen die Applikation von Wirkstoffen, z.B. Estradiol zusammen mit einem bestimmten Enhancer (hier Glycerinmonooleat) in einer beliebigen TTS-Matrix in beliebiger Konzentration beansprucht wird. Leider ist nach dem Stand der Technik auch mit solchen TTS keine befriedigende Therapie möglich, da entweder die ausgewählten Enhancer zu schlecht hautverträglich sind oder die TTS wegen des noch immer zu geringen Flusses durch die Haut unakzeptabel große Flächen aufweisen müssen. Ein weiteres Problem stellt die Tatsache dar, daß zahlreiche Rezepturvarianten solcher Systeme zur Rekristallisation aus scheinbar unvorhersehbarer Ursache neigen ud dabei eine großen Teil ihrer therapeutischen Aktivität verlieren.

Dem Stand der Technik entsprechende Konzentrationsangaben finden sich z.B. in GB 2 138 286 (zwischen 0,01% Estradiol und der Sättigungslöslichkeit im Lösungsmittel), in EP 0 421 454 (0,5% bis 10% im Polymer) oder in BE 899 444 (zwischen 20% und Sättigung).

Aufgabe dieser Erfindung ist es daher, ein akzeptabel dünnes und von der Fläche her kleines TTS bereitzustellen, das keine leichtflüchtigen Enhancer enthält, während der Lagerzeit keine Aktivitätsverluste durch Rekristallisation erleidet und dennoch eine hohe flächenbezogene Flußrate (mg/cm²/h) auf der Haut aufweist.

Diese Aufgabe wird erfindungsgemäß bei einem transdermalen therapeutischen System, enthaltend den Wirkstoff 17-β-Estradiol und gegebenenfalls weitere Wirkstoffe, mit geschichtetem Aufbau aus einer im wesentlichen feuchtigkeitsundurchlässigen Rückschicht, einer oder mehrerer Matrixschichten und gegebenenfalls einer Klebschicht, dadurch gelöst, daß die Konzentration des gelösten Estradiols in allen Matrixschichten und gegebenenfalls in der Klebeschicht zwischen seiner Sättigungskonzentration im trockenen Zustand und seiner Sättigungskonzentration im feuchten Zustand liegt, wobei unter dem Begriff "trockener Zustand" verstanden wird, daß das Grundmaterial im Gleichgewicht mit einer Gasphase mit weniger als 10% relativer Luftfeuchtigkeit steht und unter dem Begriff "feuchter Zustand", daß das Grundmaterial im Gleichgewicht zu einer Gasphase mit mehr als 90% relativer Luftfeuchtigkeit steht.

Weitere wertvolle Eigenschaften des transdermalen therapeutischen Systems ergeben sich durch dessen Ausbildung entsprechend den Unteransprüchen, wobei es während der Lagerung vorteilhaft eine Gleichgewichtsfeuchte bis zu 40% relativer Luftfeuchtigkeit aufweist. Dabei weisen überraschend alle Matrixschichten und bei Vorhandensein einer Klebschicht diese im Feuchtegleichgewicht mit bis zu 10% relativer Luftfeuchtigkeit eine um mindestens 50% höhere Löslichkeit für den Wirkstoff 17-β-Estradiol auf, als im Feuchtegleichgewicht mit mindestens 90% relativer Luftfeuchtigkeit. Es ist von Vorteil, wenn alle Matrixschichten und bei Vorhandensein einer Klebschicht diese aus einem Acrylsäureestercopolymer mit einer Löslichkeit für den Wirkstoff 17-β-Estradiol zwischen 0,2 und 2,0 (g/g), bestimmt im Feuchtegleichgewicht mit 10% relativer Luftfeuchtigkeit bestehen.
Zweckmäßig enthält eine oder mehrere der Matrixschichten oder bei Vorhandensein einer Klebschicht diese einen oder mehrere Stoffe aus der Gruppe der Tackifier, hautpermeationsverbessernde Zusätze, Füllstoffe, Löslichkeitsverbesserer oder in Wasser quellbare Zusatzstoffe.
Dabei können jeweils in einer oder mehreren Schichten der Matrix und/oder einer Klebschicht folgende Komponenten enthalten sein:
Als Bestandteile des Haftklebers klebrigmachende Harze wie Kolophonium und dessen Derivate, Polyterpenharze aus α oder β-Pinen, aliphatische, aromatische oder alkylaromatische Kohlenwasserstoffharze, Melamin-Formaldehyd-Harze, Phenolharze, Hydroabietylalkohol und deren Gemische.

Als Füllstoffe Carbonate, Phosphate, Silicate, Sulfate und Oxide der Erdalkalimetalle, Zinkoxid, Siliciumoxide, Cellulose und ihre Derivate, Talkum oder Titandioxid, aber auch schwerlösliche Zucker (-derivate), wie zum Beispiel Lactose oder Stärkederivate wie Cyclodextrine.

Als löslichkeitsverbessernde Zusätze bzw. Hautpermeationsverbesserer Acetylaceton, Acetyltributylcitrat, Acetyltriethylcitrat, Avocadoöl, Baumwollsaatöl, Benzylalkohol, Butylstearat, Cetyllactat, Cetylpalmitat, Cetylstearat, Cetylstearylalkohol/Cetylalkohol, Chlorbutanol, Cineol, Decylmethylsulfoxid, Decyloleat, Dibutylphthalat, Diethylenglycolmonoethylether, Diethylphthalat, Diethylsebacat, Diisopropyladipat, Dimethylphthalat, Dimethylsulfoxid, Dioctyladipat, Dipropylenglykol, Glycerinmonooleat, Glycerinmonostearat, Stearylalkohol, Erdnußöl, Ethyllactat, Ethyllinolat, Ethyl-(9,12,15)-linolenat, Eugenol, Farnesol, Glycerin, Glycerinacetylester, Glycerinstearat, Glycoldistearat, Glyoxal, Hexadecanol, Hexylenglykol, Vaseline, Petrolatum, Isobutylstearat, Isocetylstearat, Isodecyloleat, Isopropyllanolat, Isopropylmyristat/Isopropylpalmitat, Isopropylstearat, Isostearylneopentanoat, Laurinsäurediethanolamid, Limonen, Linolensäure, Linolsäurediethanolamid, Mandelöl, Menthol, Minzöl, Myristyllactat, Myristylmyristat, Myristylstearat, m-Tolylacetat, Nelkenöl, Octyldodecanol, Octylpalmitat, Octylstearat, Ölsäurediethanolamid, Oleylalkohol, Oleyloleat, Olivenöl, Paraffin, flüssig = mineral oil, Petroleum, Pfefferminzöl, Phenylethylalkohol, Isostearinsäure, Octansäure, Propylencarbonat, Propylenglykol, Rizinusöl/hydriert/raffiniert, Safloröl, Squalan, Squalen, Triacetin, Glycerintriacetat, Triethylcitrat, Undecylensäure, Propylenglykol, Propandiol, 1,3-Butylenglykol, (+)-Fenchon, Ammoniumlaurylethersulfat, Cholsäure, Cholesterol, Kaliumstearat, Lecithin, Glycerolhydroxystearat, Mono- und iglyceride von Speisefettsäuren, Natriumcaprylat, Natriumlaurylethersulfat, Na-/K-salze der Speisefettsäuren, Na.-laurylsulfat, PEG-(2)-stearat, Natriumsulfosuccinat, Polyglycerinester der Speisefettsäuren, Polyoxyethylenalkylether, Cetomacrogol, Polyoxyethylenfettsäuresorbitanester, Propylenglycolstearat, Sorbitanfettsäureester, Stearinsäurediethanolamid.

Als in Wasser quellbare Zusätze wie Stärke und ihre Derivate, Agar-Agar, Alginsäure, Arabinogalactan, Galactomannan, Cellulose und ihre Derivate, Carrageen, Dextran, Traganth und viele andere Gummen pflanzlicher Herkunft, sowie in Wasser lösliche oder quellbare, synthetische Polymere wie Polyvinylpyrrolidon, Polyvinylalkohol, Polyacrylsäure oder Polyacrylamid, um nur einige zu nennen. Polypeptide wie Gelatine, Albumin, Kollagen oder Eiklar.

Als Kristallisationsverzögerer Phthalsäureester, Adipinsäureester, Mono-, Di- und Triglyceride, Ester höherer Fettsäuren, langkettige Alkohole und deren Derivate, Derivate des Nonylphenol bzw. des Octylphenol, Derivate von Fettsäuren, Derivate des Sorbit und des Mannit, nichtionogene Tenside, Polyoxyethylenalkylether, Derivate des Rizinusöls, Sitosterin und Polyvinylpyrrolidon sowie weitere dem Fachmann bekannte Stoffe.

Das erfindungsgemäße Prinzip tritt in vergleichbarer Weise auch bei anderen Grundmaterialien für Transdermale Therapeutische Systeme auf. Hierzu ist wichtig vor allem die korrekte Einstellung der Estradiol-Konzentration. Diese muß höher sein als die Sättigungskonzentration im feuchten Zustand, aber niedriger als die Sättigungskonzentration unter trockenen Bedingungen. Zur Herstellung erfindungsgemäßer Systeme sind also vorher experimentelle Löslichkeitsbestimmungen erforderlich.
Da die Wasseraufnahmekapazität von Polymeren höchst unterschiedlich und auch temperaturabhängig ist, würde eine Beschreibung der Zustände "feucht" und "trocken" durch absolute Konzentrationsangaben von Wasser im Grundmaterial die Verhältnisse nicht präzise genug beschreiben. Es ist vielmehr notwendig, den in der Pharmazie üblichen Begriff der Äquivalentfeuchte zu Hilfe zu nehmen. Damit ist die Feuchte bezeichnet, die ein Material im Gleichgewicht mit einer voreingestellten relativen Luftfeuchtigkeit der Umgebung annimmt. Zweckmäßig wird die Estradiol-Löslichkeitsbestimmung daher in sogenannten Hygrostaten durchgeführt, in denen ein Hilfsmaterial bekannter Äquivalentfeuchte (Salzlösungen, Silicagel, Phosphorpentoxid etc.) gemeinsam mit der Probe in einem nach außen abgeschlossenen Behälter über einige Zeit (Tage bis Wochen) gemeinsam gelagert wird. Im anliegenden Beispiel 1 wurden praxisnahe Varianten solcher Hygrostaten verwendet, mit deren Hilfe Äquivalentfeuchten von etwa 0 bis 1%, sicher aber unter 10% relativer Luftfeuchtigkeit - trockener Zustand - (Kieselgel) bzw. etwa 97 bis 98 % - feuchter Zustand - (sogenannte physiologische Kochsalzlösung) eingesetzt wurden.

Die bei diesen Versuchen anzuwendende Methode der Löslichkeitsbestimmung ist vom Fachmann dem jeweiligen Problem anzupassen. Eine einfache Möglichkeit ist die in Beispiel 1 geschilderte. Hier wurden Testformulierungen mit verschieden hohen Wirkstoffbeladungen angefertigt und unter feuchtekontrollierten Bedingungen gelagert. Anschließend wird lediglich visuell oder mikroskopisch entschieden, ob der Wirkstoff nun vollständig gelöst oder aber ausgefällt vorliegt.

Die für den Herstell- und Lagerungsprozeß vorteilhafte Äquivalentfeuchte unter 40% relativer Luftfeuchtigkeit kann außer durch Verwendung des Hygrostaten-Prinzips auch einfach durch hinlängliche Abschirmung (Verpackung) des Gutes von der Umgebungsluft erreicht werden.
Ein Verfahren zur Herstellung erfindungsgemäßer TTS ist entsprechend den Merkmalen der Ansprüche 6 bis 8 vorgesehen.

### Beispiel 1:

Zur Bestimmung der Beeinflussung der Löslichkeit von 17-β-Estradiol durch unterschiedliche Lagerfeuchte wurden zunächst die folgenden wirkstoffhaltigen klebrigen Schichten herangezogen (Schichtdicke übereinstimmend ca. 60 Mikrometer):
- A: Acrylatpolymer mit 0,3%: Estradiol
- B: Acrylatpolymer mit 0,45%: Estradiol
- C: Acrylatpolymer mit 0,6%: Estradiol
- D: Acrylatpolymer mit 0,8%: Estradiol
- E: Acrylatpolymer mit 1,0%: Estradiol
- F: Acrylatpolymer mit 1,3%: Estradiol
- G: Acrylatpolymer mit 2,0%: Estradiol
- H: Acrylatpolymer mit 3,0%: Estradiol

Die Schichten wurden zum Schutz der Oberflächen zwischen eine 15 Mikrometer dicke PETP-Membran und einen Glas-Objektträger (76 x 26 mm) luftblasenfrei kaschiert.

Die Muster wurden mit der nötigen Feuchtereguliereinrichtung (s.u.) in siegelfähigen Verbundpackstoff (Papier/Aluminium/Ethylenvinylacetat) eingesiegelt und bei Raumtemperatur ein Jahr lang gelagert.

### Feuchteregulatoren:

1. "Feucht": eingelegter Streifen Vlies, getränkt mit ca. 1 ml 0,9% Natriumchloridlösung in Wasser
2. "Trocken": ca. 10 Körnchen Blaugel, ca. 1 g

   Da jede Probe A-H unter beiden Bedingungen gelagert wurde, ergaben sich 16 verschiedene Beobachtungsmuster.

Nach Ablauf der Lagerfrist wurden die Muster auf Ausfällungen des Wirkstoffes untersucht. Dabei wurden nur flächenhaft auftretende, mikroskopisch einwandfrei erkennbare Kristalle als "Rekristallisation" gewertet.

| Wirkstoffgehalt Befund "trocken" | Befund "feucht" |
|---|---|
| 0,3% Estradiol vollständig gelöst | vollständig gelöst |
| 0,45% Estradiol vollständig gelöst | vollständig gelöst |
| 0,6% Estradiol vollständig gelöst | Fällung |
| 0,8% Estradiol vollständig gelöst | Fällung |
| 1,0% Estradiol vollständig gelöst | Fällung |
| 1,3% Estradiol vollständig gelöst | Fällung |
| 2,0% Estradiol Fällung | Fällung |
| 3,0% Estradiol Fällung | Fällung |

Der Konzentrationsbereich, in welchem das erfindungsgemäße Prinzip genutzt werden kann, liegt also zwischen 0,6 und 1,3% Estradiol (g/g).

### Beispiel 2:

Herstellung eines erfindungsgemäßen Systems
1,0 g 17-β-Estradiol
60,0 g Cariflex ^{R} TR 1107 (Styrol-Isopren-Styrol-Blockcopolymer)
138, 0 g Foral ^{R} 85 (thermoplastisches Esterharz aus Kolophoniumderivaten)
200,0 g Benzin (Siedebereich 80 bis 100 °C)
werden bei Raumtemperatur in einem zylindrischen Glasgefäß bis zum Erreichen einer gleichmäßigen Suspension gerührt und anschließend auf einer kontinuierlich arbeitenden Beschichtungsanlage auf 100 Mikrometer dicke, siliconisierte Polyesterfolie so beschichtet, daß eine Schichtdicke von 100 g/m² (bezogen auf den lösemittelfreien Anteil) resultiert. Der Ausstrich wird jeweils 4 Minuten lang bei 40 °C, 60 °C, 80 °C und bei 120°C getrocknet. Sofort wird eine 15 Mikrometer dicke Polyesterfolie luftblasenfrei unter Walzendruck auf die getrocknete Schicht aufgelegt (zukaschiert).

Durch Stanzung mit Henkellocheisen werden transdermale Systeme von 16 cm² erhalten.

Mit dem erfindungsgemäßen transdermalen Therapie-System werden folgende vorteilhafte Wirkungen erzielt:
- gute Resorptionseigenschaften der menschlichen Haut,
- gute Hautverträglichkeit,
- ausreichender Wirkstofffluß durch die Haut bei akzeptabler Fläche (cm²) des TTS,
- keine Rekristallisation des Wirkstoffs während der Lagerung.

## Patentansprüche

1. Transdermales therapeutisches System enthaltend den Wirkstoff 17-β-Estradiol und gegebenenfalls weitere Wirkstoffe, mit geschichtetem Aufbau aus einer im wesentlichen feuchtigkeitsundurchlässigen sowie wirkstoffundurchlässigen Rückschicht, einer oder mehreren Matrixschichten und bei vorliegen von nichtklebrigen Matrixschichten einer Klebschicht, dadurch gekennzeichnet, daß die Konzentration des gelösten Estradiols in allen Matrixschichten und bei Vorhandensein einer Klebschicht in dieser zwischen seiner Sättigungskonzentration im trockenen Zustand und seiner Sättigungskonzentration im feuchten Zustand liegt, wobei unter dem Begriff "trockener Zustand" verstanden wird, daß das Grundmaterial in Gleichgewicht mit einer Gasphase mit weniger als 10% relativer Luftfeuchtigkeit steht, und unter dem Begriff "feuchter Zustand", daß das Grundmaterial im Gleichgewicht mit einer Gasphase mit mehr als 90% relativer Luftfeuchtigkeit steht, und daß eine Löslichkeit oder Verhinderung der Rekristallisation des Estradiols über einen Zeitraum von mindestens einem Jahr gewährleistet ist.

2. Transdermales therapeutisches System nach Anspruch 1, dadurch gekennzeichnet, daß es während der Herstellung und Lagerung eine Gleichgewichtsfeuchte bis zu 40% relativer Luftfeuchtigkeit aufweist.

3. Transdermales therapeutisches System nach Anspruch 1 oder 2, dadurch gekennzeichnet, daß alle Matrixschichten und bei Vorhandensein einer Klebschicht diese im Feuchtegleichgewicht mit bis zu 10% relativer Luftfeuchtigkeit eine um mindestens 50% höhere Löslichkeit für den Wirkstoff 17-β-Estradiol aufweist als im Feuchtegleichgewicht mit mindestens 90% relativer Luftfeuchtigkeit.

4. Transdermales therapeutisches System nach Anspruch 3, dadurch gekennzeichnet, daß alle Matrixschichten und bei Vorhandensein einer Klebschicht diese aus einem Acrylsäureestercopolymer mit einer Löslichkeit für den Wirkstoff 17-β-Estradiol zwischen 0,2 und 2,0% (g/g), bestimmt im Feuchtegleichgewicht mit 10% relativer Luftfeuchtigkeit.

5. Transdermales therapeutisches System nach einem oder mehreren der vorstehenden Ansprüche, dadurch gekennzeichnet, daß eine oder mehrere Matrixschichten oder bei Vorhandensein einer Klebschicht diese einen oder mehrere Stoffe aus der Gruppe der Tackifier, hautpermeationsverbessernde Zusätze, Füllstoffe, Löslichkeitsverbesserer oder in Wasser quellbare Zusatzstoffe enthält.

6. Verfahren zur Herstellung des transdermalen therapeutischen Systems nach den Ansprüchen 1 bis 5, dadurch gekennzeichnet, daß bei Raumtemperatur der Wirkstoff zusammen mit Bestandteilen der Matrixschicht bzw. der Klebschicht sowie mit einem oder mehreren Zusatzstoffen aus der Gruppe der Tackifier, hautpermeationsverbessernde Zusätze, Füllstoffe, Löslichkeitsverbesserer und/oder in Wasser quellbare Zusatzstoffe bis zum Erreichen einer gleichmäßigen Suspension gerührt und anschließend auf eine dehäsiv ausgerüstete Folie so beschichtet werden, daß eine vergleichsweise dünne Schichtdicke resultiert, worauf der Aufstrich jeweils einige Minuten lang bei stufenweise gesteigerten Temperaturen, zuletzt bei ca. 120 °C, getrocknet wird und auf die getrocknete Schicht unverzüglich eine Abdeckfolie luftblasenfrei unter Walzendruck aufgelegt und diese damit zukaschiert wird, wonach schließlich einzelne transdermale Systeme von definierter Fläche ausgestanzt werden.

7. Verfahren nach Anspruch 6, dadurch gekennzeichnet, daß die Beschichtung der dehäsiv ausgerüsteten Folie mit einer Beschichtungsmasse von ca. 100g/m² bezogen auf den lösungsmittelfreien Anteil aufgetragen wird.

8. Verfahren nach Anspruch 6 oder 7, dadurch gekennzeichnet, daß die Trocknung der Schicht jeweils ca. 4 Minuten lang bei 40 °C, 60 °C, 80 °C und 120 °C durchgeführt wird.

## Claims

1. Transdermal therapeutic system containing the active substance 17-β-estradiol and optionally further active substances, with a laminated structure comprising a backing layer which is substantially impermeable to moisture and impermeable to active substance, one or more matrix layers and, where non-adhesive matrix layers are present, an adhesive layer, characterized in that the concentration of the dissolved estradiol in all matrix layers and, where an adhesive layer is present, in the adhesive layer, lies between its saturation concentration in dry condition and its saturation concentration in moist condition, whereby the term "dry condition" is understood to mean that the base material is in equilibrium with a gas phase with less than 10% relative humidity, and the term "moist condition" is understood to mean that the base material is in equilibrium with a gas phase with more than 90% relative humidity, and that a solubility or prevention of recrystallization of the estradiol is secured over a period of at least one year.

2. Transdermal therapeutic system according to claim 1, characterized in that during manufacture and storage it has an equivalent moisture corresponding to up to 40% relative humidity.

3. Transdermal therapeutic system according to claim 1 or 2, characterized in that all matrix layers and, where an adhesive layer is present, the adhesive layer, in a moisture equilibrium with up to 10% relative humidity, have a solubility for the active substance 17-β-estradiol which is at least 50% higher than that in a moisture equilibrium with at least 90% relative humidity.

4. Transdermal therapeutic system according to claim 3, characterized in that all matrix layers and, where an adhesive layer is present, the adhesive layer consist of an acrylic acid ester copolymer having a solubility for the active substance 17-β-estradiol of between 0.2 and 2.0% (g/g), determined in a moisture equilibrium with 10% relative humidity.

5. Transdermal therapeutic system according to one or more of the preceding claims, characterized in that one or more matrix layers or, where an adhesive layer is present, the adhesive layer contain one or more substances from the group of tackifiers, skin permeation-enhancing additives, filling agents, solubility enhancers or water-swellable additives.

6. Process for the production of the transdermal therapeutic system according to claims 1 to 5, characterized in that at room temperature the active substance is stirred together with components of the matrix layer or adhesive layer, respectively, as well as with one or more additives from the group of tackifiers, skin permeation-enhancing additives, filling agents, solubility enhancers and/or water-swellable additives until a homogeneous suspension is obtained and are subsequently coated onto a sheet which has been rendered dehesive, such that a comparatively thin layer thickness results, subsequent to which the coating is dried at stepwise increased temperatures for some minutes at each temperature, ultimately at 120 °C, and a covering sheet is immediately applied onto the dried layer, such that no air bubbles occur, employing roll pressure, and said layer is laminated therewith covering the same, whereafter, finally, individual transdermal systems of defined area are punched out.

7. Process according to claim 6, characterized in that the coating of the sheet, which sheet has been rendered dehesive, is applied with a coating mass of 100 g/m², relative to the solvent-free portion.

8. Process according to claim 6 or 7, characterized in that drying of the layer is carried out at 40 °C, 60 °C, 80 °C and 120 °C, for 4 minutes at each temperature.

## Revendications

1. Système thérapeutique transdermique contenant le principe actif 17-β-oestradiol et éventuellement d'autres principes actifs, avec une structure en plusieurs couches constituée d'une couche dorsale essentiellement imperméable à l'humidité et imperméable aux principes actifs, d'une ou de plusieurs couches matricielles et en cas de couches matricielles non-adhésives, d'une couche adhésive, caractérisé en ce que la concentration de l'oestradiol solubilisé dans toutes les couches matricielles et dans la couche de colle, en cas de présence de celle-ci, se trouve entre sa concentration de saturation à l'état sec et sa concentration de saturation à l'état humide, le terme "état sec" signifiant que le matériau de base est en équilibre avec une phase gazeuse de moins de 10 % d'humidité relative de l'air, et le terme "état humide" signifiant que le matériau de base est en équilibre avec une phase gazeuse de plus de 90 % d'humidité relative de l'air, et en ce qu'on garantit la solubilisation ou la non-recristallisation de l'oestradiol pendant une durée d'au moins un an.

2. Système thérapeutique transdermique selon la revendication 1, caractérisé en ce qu'il présente pendant la préparation et pendant le stockage une humidité d'équilibre jusqu'à 40 % d'humidité relative de l'air.

3. Système thérapeutique transdermique selon la revendication 1 ou 2, caractérisé en ce que toutes les couches matricielles et la couche de code, en cas de présence de celle-ci, présentent à l'équilibre d'humidité avec 10 % d'humidité relative de l'air, une solubilité supérieure d'au moins 50 % pour le principe actif 17-β-oestradiol qu'à l'équilibre d'humidité avec au moins 90 % d'humidité relative de l'air.

4. Système thérapeutique transdermique selon la revendication 3, caractérisé en ce que toutes les couches matricielles et la couche de code, en cas de présence de celle-ci, sont constituées d'un copolymère d'ester d'acide acrylique avec une solubilité pour le principe actif 17-β-oestradiol entre 0,2 et 2,0 % en poids (w/w), déterminée à l'équilibre d'humidité avec 10 % d'humidité relative de l'air.

5. Système thérapeutique transdermique selon une ou plusieurs des revendications précédentes, caractérisé en ce qu'une ou plusieurs couches matricielles ou une couche de colle, en cas de présence de celle-ci, contient une ou plusieurs substances du groupe des agents adhésifs (tackifier), des additifs améliorant la perméation de la peau, des agents de charge, des agents améliorant la solubilité ou des additifs gonflables dans l'eau.

6. Procédé de production du système thérapeutique transdermique selon les revendications 1 à 5, caractérisé en ce qu'on agite à température ambiante le principe actif ensemble avec les constituants de la couche matricielle, respectivement de la couche de colle, de même qu'avec un ou plusieurs additifs du groupe des agents adhésifs (tackifier), des additifs améliorant la perméation de la peau, des agents de charge, des agents améliorant la solubilité et/ou des additifs gonflables dans l'eau, jusqu'à l'obtention d'une suspension homogène et en ce qu'on applique ensuite un revêtement sur un film anti-adhésif de telle manière qu'il en résulte une épaisseur de couche relativement faible, l'enduit étant séché chaque fois pendant quelques minutes à des températures croissantes par paliers, finalement à environ 120 °C, et sur la couche séchée étant appliquée immédiatement un film de recouvrement à l'exclusion de bulles d'air par la pression d'un rouleau et celle-ci étant ainsi contre-plaquée, étape après laquelle des systèmes transdermiques individuels d'une surface définie sont découpés.

7. Procédé selon la revendication 6, caractérisé en ce que le revêtement du film anti-adhésif est appliqué avec une masse de revêtement d'environ 100 g/m², par rapport à la fraction exempte de solvant.

8. Procédé selon la revendication 6 ou 7, caractérisé en ce que le séchage de la couche est effectué chaque fois pendant environ 4 minutes à 40 °C, 60 °C, 80 °C et 120 °C.
